# EUROPÄISCHE PATENTSCHRIFT

(11) **EP 0 481 406 B1**
(45) Veröffentlichungstag und Bekanntmachung des Hinweises auf die Patenterteilung: **03.01.1996**
(21) Anmeldenummer: 91117495.1
(22) Anmeldetag: 14.10.1991
(51) Int. Cl.: C07C 6/04, C07C 21/18

(54) **Verfahren zur Metathese teilweise fluorierter Kohlenwasserstoffe**
Process for the metathesis of partially fluorinated hydrocarbons
Procédé de métathèse d'hydrocarbures partiellement fluorés

(30) Priorität: 17.10.1990 DE 4032896
(43) Veröffentlichungstag der Anmeldung: 22.04.1992
(73) Patentinhaber: HOECHST AKTIENGESELLSCHAFT, D-65926 Frankfurt am Main (DE)
(72) Erfinder: von Werner, Konrad, Dr., W-8268 Garching (DE); Weiss, Karin, Dr., W-8580 Bayreuth (DE)

(56) Entgegenhaltungen:
- EP-A- 0 191 675
- EP-A- 0 218 138
- EP-A- 0 276 096
- EP-A- 0 373 488

## Beschreibung

Die Erfindung betrifft ein Verfahren zur Metathese teilweise fluorierter Kohlenwasserstoffe, die eine Doppelbindung aufweisen.

Die Disproportionierung olefinischer Verbindungen, die funktionelle Gruppen enthalten können, unter Einwirkung von Wärme und/oder Katalysatoren, die in der Regel Übergangsmetalle enthalten, wobei Verbindungen gebildet werden, die ein höheres und niedrigeres Molekulargewicht als die Ausgangssubstanz aufweisen, seit 1967 mit "Metathese" bezeichnet, ist über 25 Jahre bekannt. Analog der Metathese von olefinischen Verbindungen verläuft die Ringöffnungspolymerisation von Cycloolefinen.

Ausführliche Beschreibungen enthalten die Bücher "Olefin Metathesis and Ring-opening Polymerization of Cycloolefins" von V. Dragutan, A. T. Balaban und M. Dimonic, Editura Academici/Bukarest, 1981 beziehungsweise John Wiley + Sons/Chichester-New York, 1985 und "Olefin Metathesis" von K. J. Ivin, Academic Press/London-New York, 1983. Für die Olefin-Metathese wurden heterogene und homogene Katalysatoren eingesetzt, die Verbindungen von Übergangsmetallen, vor allem von Molybdän, Wolfram und Rhenium, enthalten. Einfache binäre und ternäre Katalysatorsysteme mit Metallverbindungen, wie Oxiden, Sulfiden und (Halogen)Salzen, sowie auch Metallkomplexverbindungen, die beispielsweise organische Reste sowie Liganden, wie CO, oder tertiäre Phosphine enthalten, sind bekannt. Als Träger für heterogene Katalysatoren werden beispielsweise Al₂O₃ und SiO₂ verwendet.

Als funktionelle Gruppen, die die Olefine tragen, sind beispielsweise Cl, Br, Alkoxyalkyl-, Keto-, Amino-, Alkoxycarbonyl-, Carboxyl- und Cyano-Gruppen verwendet worden. Auch Co-Metathesen zweier verschiedener olefinischer Verbindungen oder einer olefinischen mit einer cycloolefinischen sind bekannt.

K. Weiß und M. Denzner, J. of Organometallic Chemistry, 355 (1988) Seiten 273 bis 280 beschreiben als für die Metathese von Alkenen geeignete heterogene Katalysatoren Reaktionsprodukte von Wolfram(VI)-Carbin-Komplexverbindungen mit Oberflächen-Chrom(II)-Verbindungen auf Kieselgel.

Reaktionsprodukte von Wolfram(VI)-Carbin-Komplexverbindungen nur mit Kieselgel sind als Metathese-Katalysatoren von K. Weiß und G. Lößel, Angew. Chemie, 101 (1989) Seiten 75 bis 77 [C.A., Vol. 110 (1989), 134664t] beschrieben.

Eine Reihe Veröffentlichungen von W. J. Feast und Mitarbeitern: Journ. of Molecular Catalysis, 8 (1980) Seiten 277 bis 296; Polymer, 20 (1979) Seiten 1182 bis 1183 sowie 27 (1986) Seiten 1281 bis 1288 und Seiten 1296 bis 1303 befassen sich mit der ringöffnenden Polymerisation von Kohlenwasserstoff-Ringsystemen, die entfernt von der C=C-Doppelbindung mit Fluor und/oder Trifluormethylgruppen substituiert sind, wobei unter anderem homogene, Wolfram enthaltende Katalysatoren verwendet werden.

Aus der EP 0 373 488 A2 ist die Metathese von Olefinen mit Katalysatoren bekannt, die aus oxidischen Trägermaterialien bestehen, auf die Verbindungen der allgemeinen Formel R¹ₐRe_{b}O_{c} aufgebracht sind, worin a 1 bis 6, b 1 bis 4 und c 1 bis 14 sind und die Summe von a, b und c so ist, daß sie der 5- bis 7-Wertigkeit des Rheniums gerecht wird mit der Maßgabe, daß c nicht größer als 3·b ist, und worin R¹ einen Alkylrest mit 1 bis 9 C-Atomen, einen Cycloalkylrest mit 5 bis 10 C-Atomen oder einen Aralkylrest mit 7 bis 9 C-Atomen darstellt, wobei R¹ wenigstens teilweise fluoriert sein kann, die Verbindungen nicht mehr als drei Gruppen mit mehr als 6 C-Atomen je Rheniumatom enthalten und an das C-Atom in α-Stellung noch wenigstens ein Wasserstoffatom gebunden ist. Zur Erzielung von guten Ausbeuten benötigt man jedoch, bezogen auf das Alken, relativ hohe Katalysatormengen und lange Reaktionszeiten.

Aufgabe der Erfindung ist es, ein Verfahren bereitszustellen, das es ermöglicht, aus Verbindungen, die einen fluorierten Alkylrest sowie mindestens eine -CH₂-CH=CH-Gruppe enthalten, neue längerkettige Verbindungen herzustellen, die mindestens eine -CH₂-CH=CH-CH₂-Gruppe und mindestens einen fluorierten Alkylrest enthalten.

Diese Aufgabe wird gelöst durch ein Verfahren zur Umsetzung von gegebenenfalls substituierten Kohlenwasserstoffen, die mindestens eine Doppelbindung aufweisen, mit Wolfram enthaltenden Katalysatoren unter Abspaltung von Ethylen oder einem Ethylenderivat und Bildung längerkettiger substituierter Kohlenwasserstoffe, die mindestens eine mittelständige Doppelbindung aufweisen, bei 10 bis 200 °C, falls erforderlich in Gegenwart eines inerten Lösungsmittels, unter normalem Atmosphärendruck, vermindertem Druck oder dem autogenen Druck der Reaktionsmischung, das dadurch gekennzeichnet ist, daß zur Umsetzung mindestens ein Alken der Formeln

X-R_{f}-(CH₂)ₙ-CH=CH-Z (I)

oder

CH₂F-(CH₂)ₘ-CH=CH-Z (II)

eingesetzt wird, in denen bedeuten
- R_{f}: ein geradkettiger oder verzweigter oder ringförmiger Perfluoralkylenrest mit 1 bis 18 C-Atomen,
- m und n: je eine ganze Zahl von 1 bis 10,
- X: = F, H, Cl, Alkyl mit 1 bis 6 C-Atomen oder Aryl mit 6 bis 10 C-Atomen und
- Z: = H, Alkyl mit 1 bis 10 C-Atomen, Arylalkyl mit 7 bis 11 C-Atomen, den Rest -(CH₂)ₙR_{f}X oder den Rest -(CH₂)ₘ-CH₂F, wobei, wenn nur eine Verbindung eingesetzt wird, diese unterschiedliche Substituenten auf beiden Seiten der -CH=CH-Gruppe aufweisen muß.

Zur Durchführung des erfindungsgemäßen Verfahrens geeignete Verbindungen der Formel (I) sind beispielsweise:
(CF₃)₂CF-(CH₂)₂-CH=CH₂
C₆F₁₃-CH₂-CH=CH₂
C₈F₁₇-(CH₂)₂-CH=CH₂
CHF₂-(CF₂)₃-(CH₂)₂-CH=CH₂
CClF₂-CF₂-(CH₂)₃-CH=CH₂
C₂H₅-(CF₂)₂-(CH₂)₄-CH=CH₂
Phenyl-(CF₂)₂-(CH₂)₂-CH=CH₂ 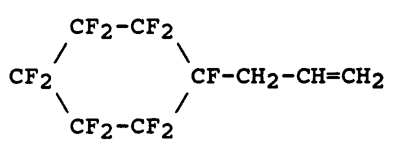
C₄F₉-(CH₂)₂-CH=CH-(CH₂)₂C₄F₉
C₆F₁₃-CH₂-CH=CH-C₄H₉
C₁₂F₂₅-(CH₂)₄-CH=CH-CH₂-Phenyl
CHF₂-(CF₂)₂-(CH₂)₂-CH=CH-(CH₂)₂-(CF₂)₂-CHF₂
Geeignete Verbindungen der Formel (II) sind beispielsweise:
CH₂F-(CH₂)₃-CH=CH₂
CH₂F-(CH₂)₆-CH=CH-C₂H₅
CH₂F-(CH₂)₈-CH=CH-CH₂-Phenyl
CH₂F-(CH₂)₂-CH=CH-(CH₂)₂-CH₂F
Es können eine oder mehrere der Verbindungen der Formel (I) und (II) eingesetzt werden. Sofern nur eine einzige Verbindung verwendet wird, muß diese verschiedene Substituenten auf beiden Seiten der -CH=CH-Gruppe aufweisen. Bevorzugt werden Verbindungen der Formel (I) eingesetzt, in denen R_{f} eine Perfluoralkylengruppe mit 2 bis 10, insbesondere mit 3 bis 8 C-Atomen ist. Ferner sind bevorzugt solche Verbindungen der Formel (I), in denen X = F ist. Wegen ihrer guten Zugänglichkeit und Reaktionsfähigkeit sind ferner Verbindungen der Formel (I) bevorzugt, in der n eine ganze Zahl von 1 bis 4, insbesondere von 1 oder 2, ist. Das gleiche gilt für m in Verbindungen der Formel (II). Der Substituent Z in der Verbindung der Formel (I) beziehungsweise in der Verbindung der Formel (II) wird vorteilhaft so gewählt, daß der Siedepunkt seiner Vinylverbindung bei normalem Atmosphärendruck unterhalb der gewählten Reaktionstemperatur liegt und der Substituent Z mindestens 2 C-Atome weniger enthält als der Substituent XR_{f}(CH₂)ₙ- in der Verbindung der Formel (I) beziehungsweise CH₂F(CH₂)ₘ- in der Verbindung der Formel (II). Bevorzugt ist der Substituent Z ein Alkylradikal mit 3 oder 4 C-Atomen und insbesondere H, Methyl oder Ethyl. Besonders gute Ergebnisse werden erhalten, wenn Verbindungen der Formel (I) eingesetzt werden, in denen X = F und Z = H ist.

Zusätzlich zu einer oder mehreren Verbindungen der Formeln (I) und (II) können nicht fluorhaltige Verbindungen der Formel

A-(CH₂)ₜ-CH=CH-E (III)

eingesetzt werden, in der bedeuten
- A =: H, -CH₂Cl, Aryl mit 6 bis 10 C-Atomen oder -CH=CH₂,
- t =: eine ganze Zahl von 1 bis 10 und
- E =: H, Alkyl mit 1 bis 10 C-Atomen oder Arylalkyl mit 7 bis 11 C-Atomen,
wobei die Bindungen bei A und E zu einem Ring geschlossen sein können.

Für das erfindungsgemäße Verfahren geeignete Verbindungen sind beispielsweise:
CH₃-(CH₂)₇-CH=CH₂
CH₂Cl-CH₂-CH=CH₂
Phenyl-CH₂-CH=CH₂
CH₂=CH-(CH₂)₂-CH=CH₂
CH₂Cl-CH₂-CH=CH-CH₂-CH₂Cl
Phenyl-CH₂-CH=CH-CH₂-Phenyl
CH₃-(CH₂)₄-CH=CH-CH₃
CH₃-(CH₂)₇-CH=CH-(CH₂)₇-CH₃ 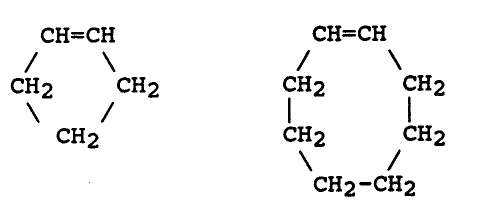
Bevorzugt werden Verbindungen verwendet, in denen der Substituent E so gewählt ist, daß der Siedepunkt seiner Vinylverbindung bei Atmosphärendruck unterhalb der gewählten Reaktionstemperatur liegt und der Substituent E mindestens 2 C-Atome weniger enthält, als der Substituent A-(CH₂)t-. Vorzugsweise ist der Substituent E ein Alkylradikal mit 3 oder 4 C-Atomen und insbesondere H, Methyl oder Ethyl. Wenn A = CH₂=CH- ist, können oligomere Produkte mit größerer Kettenlänge erhalten werden, die dadurch entstehen, daß 2 oder mehr mol der Verbindung der Formel (III) mit 1 oder 2 mol der Verbindung der Formel (I) reagieren. Vorteilhaft werden für solche Reaktionen Verbindungen der Formel (III) eingesetzt, in denen A = CH₂=CH- und E = H ist.

Bei der erfindungsgemäßen Umsetzung wird Ethylen abgespalten, wenn alle eingesetzten Verbindungen der Formeln (I), (II) und (III) eine -CH=CH₂-Gruppe aufweisen, es kann Ethylen abgespalten werden, wenn mindestens eine der eingesetzten Verbindungen der Formeln (I), (II) oder (III) eine -CH=CH₂-Gruppe enthält. Ist dies nicht der Fall, so werden Alkene abgespalten, die auf beiden Seiten der -CH=CH-Gruppe mit einem organischen Rest substituiert sind. Bei Mischungen von Ausgangssubstanzen, die sowohl die Gruppe -CH=CH₂ wie auch die beidseitig substituierte Gruppe -CH=CH- aufweisen, können auch einseitig substituierte Alkene -CH=CH₂ abgespalten werden.

Die erfindungsgemäße Umsetzung findet in Gegenwart Wolfram enthaltender Katalysatoren statt. Gut geeignet sind hierzu beispielsweise Komplexverbindungen der Formel 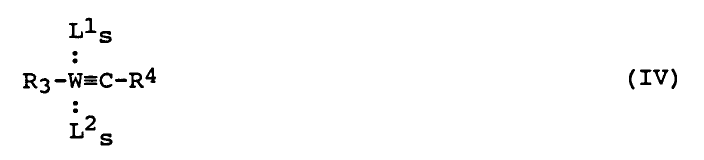
in der bedeuten
- R =: Cl, -CH₂-C(CH₃)₃, -O-C(CH₃)₃, -S-C(CH₃)₃ oder -NG₂, worin G eine Alkylgruppe mit 1 bis 4 C-Atomen oder der Benzylrest ist,
- R⁴ =: eine geradkettige, verzweigte oder cyclische Alkylgruppe mit 1 bis 6 C-Atomen, eine Trialkylsilylgruppe, deren Alkylreste je 1 bis 3 C-Atome enthalten, eine Benzyl-, Phenyl-, Tolyl- oder Naphthylgruppe,
- L¹ =: ein Trialkylphosphin, Trialkylphosphinoxid oder Ethylenglykoldialkylether, deren Alkylgruppen 1 bis 4 C-Atome aufweisen,
- L² =: ein Trialkylphosphin, Trialkylphosphinoxid, Trialkylphosphoniumchlorid oder Tetraalkylammoniumchlorid, deren Alkylgruppen 1 bis 4 C-Atome aufweisen, mit der Maßgabe, daß R = Cl ist, wenn L² ein Trialkylphosphoniumchlorid oder Tetraalkylammoniumchlorid ist, und
- s =: Null oder 1.

Beispiele für solche Wolfram enthaltenden Katalysatoren sind:
(CH₃OCH₂CH₂OCH₃)Cl₃W≡C-C(CH₃)₃
[(CH₃)₃C-CH₂-]₃W≡C-C(CH₃)₃
[(CH₃)₃C-O-]₃W≡C-C(CH₃)₃
[(CH₃)₃C-S-]₃W≡C-C(CH₃)₃
[(CH₃)₂N-]₃W≡C-C(CH₃)₃
[(CH₃)₃P]Cl₃W≡C-C(CH₃)₃
[(C₂H₅)₃P]Cl₃W≡C-C(CH₃)₃
[(C₂H₅)₃PO]Cl₃W≡C-C(CH₃)₃
[(C₂H₅)₃P][(C₂H₅)₃PO]Cl₃W≡C-C(CH₃)₃
[(C₂H₅)₃PH]⁺ [Cl₄W≡C-C(CH₃)₃]⁻
[(C₂H₅)₄N]⁺ [Cl₄W≡C-C(CH₃)₃]⁻ 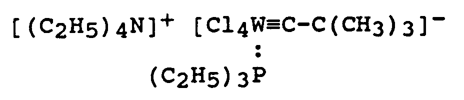
(CH₃OCH₂CH₂OCH₃)Cl₃W≡C-CH₃ 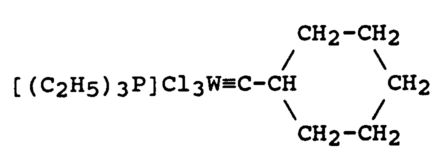
[(CH₃)₃P]Cl₃W≡C-CH₂-Phenyl
[(CH₃)₃C-CH₂]₃-W≡C-Phenyl
[(CH₃)₃C-CH₂]₃-W≡C-Si(CH₃)₃
Die Herstellung solcher Wolfram-Komplexverbindungen ist beschrieben von R. R. Schrock, D. N. Clark, I. Sancho, I. H. Wengrovius, S. M. Rocklage und S. F. Pedersen in "Organometallics", 1 (1982), Seiten 1 645 bis 1 650.

Die genannten Wolfram-Komplexverbindungen können als solche zu den flüssigen Verbindungen der Formeln (I) und/oder (II) sowie auch zu deren flüssigen Mischungen mit einer oder mehreren Verbindung(en) der Formel (III) gegeben werden. Vorzugsweise werden jedoch Lösungsmittel zugesetzt. Geeignet sind hier flüssige, gesättigte Kohlenwasserstoffe, wie Hexan, Heptan, Octan, deren Isomeren-Gemische sowie deren Chlor- beziehungsweise Fluor-Substitutionsprodukte, wie Propylchlorid, Butylchlorid, Cyclohexylchlorid, Dichlormethan, Dichlorethan, Dichlorpropan, Chloroform, Trichlorethan, Tetrachlormethan, Trichlorfluormethan und Trichlortrifluorethan.

Neben den Wolfram-Verbindungen können zusätzlich als Cokatalysatoren Metallalkyle, wie Sn(CH₃)₄, Alkylmetallhalogenide, wie (C₂H₅)₂AlCl, Metallhalogenide, wie SnCl₄, TiCl₄, und aliphatische Alkohole oder Phenole eingesetzt werden.

Ferner sind gut geeignete Katalysatoren solche, die aufgebaut sind aus einem festen Träger, der im wesentlichen aus Siliciumdioxid, Aluminiumoxid oder Mischungen dieser beiden Oxide besteht, an dem über Sauerstoffatome der genannten Oxide oder über Chrom, das an diese Sauerstoffatome gebunden ist, eine Wolfram-Komplexverbindung, die eine Bindung zwischen Wolfram und einem Kohlenstoffatom aufweist, gebunden ist.

Es wird angenommen, daß sich hierbei folgende Strukturen bilden:

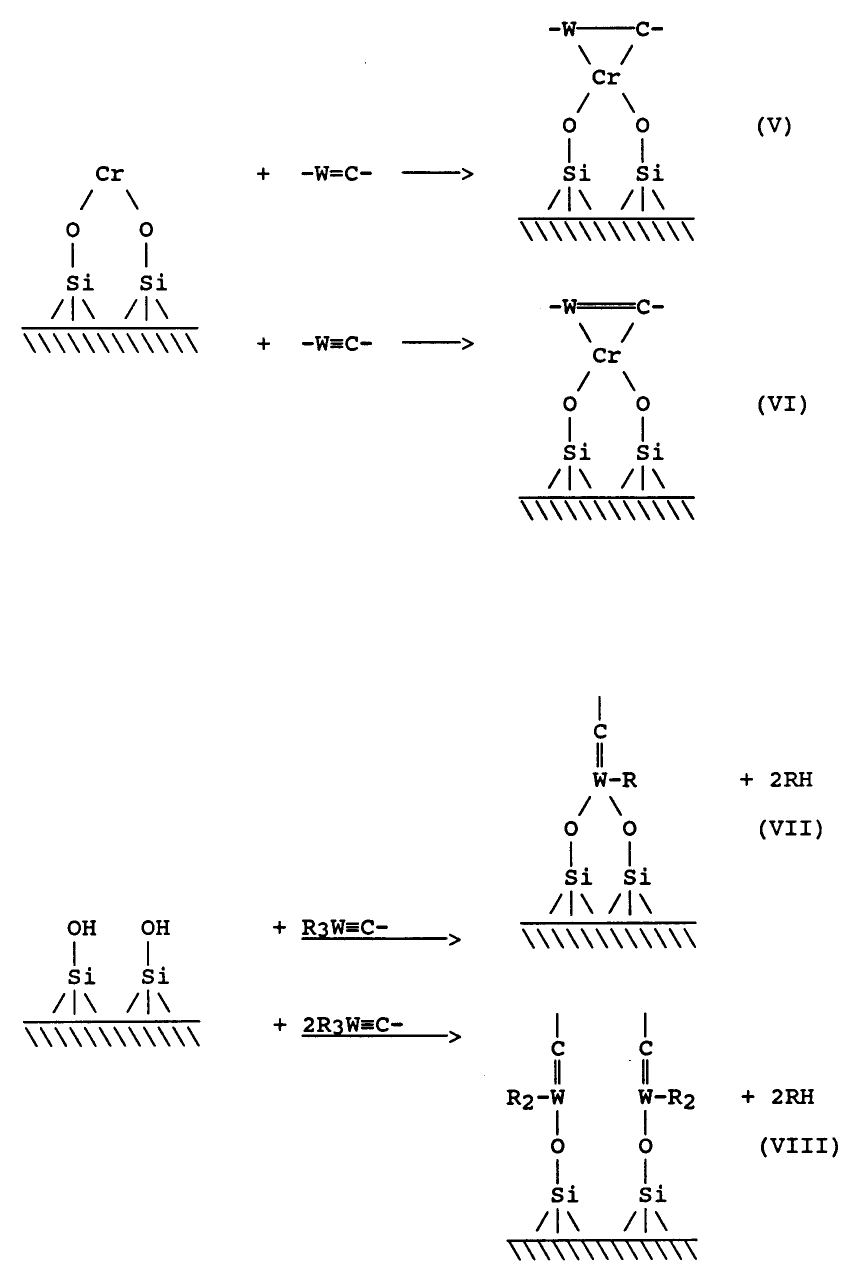

Als Wolfram-Verbindungen des Typs R₃W≡C- sind gut geeignet die weiter oben näher beschriebenen Verbindungen der Formel (IV), die durch die Bindung an den festen Träger besser lagerfähig und häufig auch noch aktiver werden. Solche Katalysatoren sind beschrieben von K. Weiß und G. Lößel in "Angewandte Chemie", 101 (1989), Seiten 75 bis 77.

Als Wolfram-Verbindungen des Typs -W≡C- sind beispielsweise Verbindungen folgender Formel geeignet:

(CO)_{y}QW≡C-R⁵ (IX)

worin bedeuten
- Q: = Cl, Br, I und
- y: = 4
oder
- Q: = Cyclopentadienyl, Phenyl, Naphthyl und
- y: = 2 sowie
- R⁵: = eine geradkettige, verzweigte oder cyclische Alkylgruppe mit 1 bis 6 C-Atomen, Phenyl, Naphthyl oder -NT₂, worin T eine Alkylgruppe mit 1 bis 4 C-Atomen ist.

Beispiele für geeignete Verbindungen sind:
(CO)₄ClW≡C-Phenyl
(CO)₄BrW≡C-Phenyl
(CO)₂-Cyclopentadienyl-W≡C-Phenyl
(CO)₂-Phenyl-W≡C-C(CH₃)
(CO)₄ClW≡C-C₂H₅
(CO)₂-Cyclopentadienyl-W≡C-N(CH₃)₂
deren Anlagerungsprodukte an die oben näher erläuterten festen Träger, siehe Formel (VI), sind beschrieben von K. Weiß und M. Denzner in "Journal of Organometallic Chemistry", 355 (1988), Seiten 273 bis 280.

Ferner sind als Wolfram-Verbindungen des Typs -W=C-beispielsweise Verbindungen folgender Formel geeignet

(CO)₅W=CR¹R² (X)

in der bedeuten
- R¹ =: Alkyl mit 1 bis 10 C-Atomen, Benzyl, Phenyl oder Naphthyl, wobei der aromatische Ring dieser Reste seinerseits mit 1 bis 3 Alkylgruppen substituiert sein kann, die ihrerseits zusammen 1 bis 12 C-Atome aufweisen, und
- R² =: Alkyl mit 1 bis 10 C-Atomen, Alkoxy mit 1 bis 6 C-Atomen, Benzyl, Phenyl oder Naphthyl, Phenoxy oder Naphthoxy, wobei der aromatische Ring dieser Reste seinerseits mit 1 bis 3 Alkylgruppen subsitituiert sein kann, die ihrerseits zusammen 1 bis 12 C-Atome aufweisen.

Bei der Reaktion der Verbindungen der Formel (X) mit dem Chrom(II) enthaltenden festen Träger, der im wesentlichen aus Siliciumdioxid, Aluminiumoxid oder Mischungen dieser beiden Oxide besteht, wird CO abgespalten, so daß Verbindungen der Formel 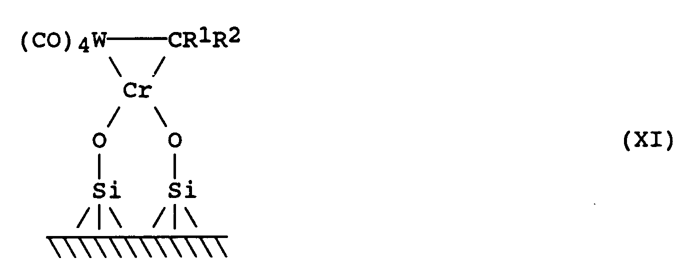
entstehen. Die Herstellung solcher fester Katalysatoren ist beschrieben von K. Weiß, W. Guthmann und S. Maisuls in "Angewandte Chemie", 100 (1988), Seiten 268 bis 270.

In den vorangegangenen Ausführungen umfaßt der Begriff "Siliciumdioxid, Aluminiumoxid oder Mischungen dieser beiden Oxide" nicht nur physikalische Mischungen, sondern auch chemisch verbundene Mischoxide beziehungsweise Alumosilicate.

Die in den vorstehenden Abschnitten beschriebenen, aus festen Trägern aufgebauten Katalysatoren können als solche verwendet werden, wie auch in kleinen Partikeln in Lösungsmitteln, beispielsweise den weiter oben beschriebenen Kohlenwasserstoffen, die chloriert oder fluoriert sein können, suspendiert. Sofern die Verbindung(en) der Formel (I) oder (II), gegebenenfalls zusammen mit einer Verbindung der Formel (III), bei der gewählten Reaktionstemperatur in der Gasphase vorliegen, können die aus festen Trägern aufgebauten Katalysatoren in feinverteilter, aufgewirbelter Form oder als Festbett angewendet werden.

Im allgemeinen werden auf 100 mol einer oder mehrerer Verbindungen der Formeln (I), (II) und (III) 0,01 bis 2 mol, vorzugsweise 0,1 bis 1 mol, Wolfram-Verbindung im Katalysator eingesetzt, jedoch kann insbesondere bei Verwendung von Katalysatoren, die aus festen Trägern aufgebaut sind, die Menge der Wolfram-Verbindung auch über 2 mol je 100 mol Verbindungen der Formeln (I), (II) und (III) betragen.

Das Mischungsverhältnis der Verbindungen der Formeln (I) oder (II) untereinander oder mit einer oder mehreren der Verbindung(en) der Formel (III) kann in weiten Grenzen schwanken. Im allgemeinen wird man je 1 mol der Verbindung der Formel (I) oder der Formel (II) 0,5 bis 3 mol, vorzugsweise 1 bis 2 mol, der Verbindung der Formel (III) verwenden.

Die erfindungsgemäße Umsetzung wird bei 10 bis 200 °C durchgeführt, unter 10 °C verläuft die Umsetzung im allgemeinen zu langsam, oberhalb 200 °C werden zuviel unerwünschte Nebenprodukte gebildet. Die Wahl einer optimalen Temperatur hängt von der thermischen Stabilität der eingesetzten Ausgangsstoffe und Katalysatoren sowie der zu erzielenden Endprodukte ab. Vorteilhaft werden Temperaturen von 30 bis 150 °C und insbesondere von 50 bis 120 °C angewendet.

Im allgemeinen wird bei normalem Atmosphärendruck oder dem autogenen Druck der Reaktionsmischung gearbeitet. Wenn das bei der erfindungsgemäßen Umsetzung abgespaltene Ethylenderivat schwerer flüchtig ist, empfiehlt sich die Anwendung verminderten Druckes und gegebenenfalls höher siedender Lösungsmittel. Die Abtrennung des abgespaltenen Ethylenderivates kann vermittels Überleiten oder Durchleiten eines Inertgases, beispielsweise Stickstoff oder Argon, durch beziehungsweise über die Reaktionsmischung beschleunigt werden. Das gasförmig abgetriebene Ethylen beziehungsweise Ethylenderivat kann entweder beispielsweise durch Verbrennen entsorgt werden oder durch Adsorption an geeignetem Medium oder Kondensation wiedergewonnen werden.

Reaktionsgemische, die ausschließlich schwer flüchtige Verbindungen enthalten, können, gegebenenfalls nach Verdünnung mit einem geeigneten Lösungsmittel, chromatographisch getrennt werden.

Das nach Abtrennung der Umsetzungsprodukte mit niedrigerem Molekulargewicht verbleibende höher molekulare Umsetzungsprodukt wird von etwa anwesendem Lösungsmittel durch fraktionierte Destillation, gegebenenfalls unter vermindertem Druck, getrennt und gereinigt. Bei Gasphasenprozessen wird das höher molekulare Umsetzungsprodukt zweckmäßig durch fraktionierte Kondensation gewonnen, das gleiche gilt für nicht umgesetzte Ausgangsstoffe, die in den Prozeß zurückgeführt werden können.

Die Zeit für die erfindungsgemäße Umsetzung kann in weiten Grenzen schwanken. Sie hängt von den Ausgangsprodukten, dem Katalysator und der Reaktionstemperatur ab. Im allgemeinen ist die Umsetzung beendet, wenn kein Ethylen oder niedermolekulares Ethylenderivat mehr abtrennbar ist. Bei diskontinuierlicher Arbeitsweise sind Reaktionszeiten von 2 bis 40 Stunden meist ausreichend.

Das Reaktionsgemisch wird während der erfindungsgemäßen Umsetzung zweckmäßig ständig gut durchmischt, insbesondere wenn ein feinteiliger fester Katalysator eingesetzt wird. Diese Durchmischung kann beispielsweise durch Rühren, Schütteln, Umpumpen, Sieden, Gasentwicklung aus der Reaktionsmischung oder Leiten eines Inertgases durch die Reaktionsmischung bewirkt werden.

Erfindungsgemäß herstellbar sind auch Verbindungen der Formel

CpF₂ₚ₊₁(CH₂)ᵣCH=CH(CH₂)ᵣ-CpF₂ₚ₊₁ (XII)

in der bedeuten
- p =: eine Zahl von 2 bis 10, vorzugsweise 3 bis 8, und
- r =: eine Zahl von 1 bis 4, vorzugsweise 1 oder 2.

Diese Verbindungen werden beispielsweise nach dem vorstehend beschriebenen neuen Verfahren hergestellt, wobei zweckmäßig ausschließlich eine Verbindung der Formel

XR_{f}-(CH₂)ₙ-CH=CH-Z (I)

eingesetzt wird, in der bedeuten
- X: = F,
- Z: = H,
- R_{f}: einen geradkettigen oder verzweigten Perfluoralkylenrest mit 2 bis 10 C-Atomen und
- n: eine ganze Zahl von 1 bis 4.

Die nach dem neuen Verfahren erhältlichen längerkettigen Verbindungen, die mindestens einen fluorierten Alkylrest und mindestens eine -CH₂-CH=CH-CH₂-Gruppe aufweisen, sind insbesondere wegen ihrer reaktionsfähigen Doppelbindung verschiedenen weiteren Reaktionen zugänglich, die zu wertvollen Substanzen führen. Wenn beispielsweise die Doppelbindung hydriert wird, entstehen Produkte, die als wärmebeständige Gleitmittel verwendet werden können; sie sind auch als Gleitwachse für sportliche Zwecke (Ski, Rodel) geeignet, wobei sie gegenüber den vollständig fluorierten Kohlenwasserstoffen noch eine gewisse Mischbarkeit mit nicht fluorierten Paraffinen aufweisen. Mit kürzeren fluorierten Alkylresten sind die Verbindungen in Mineralölen löslich und können als entsprechende Zusätze verwendet werden. Die Doppelbindung kann beispielsweise mit organischen Persäuren, wie Metachlorperoxybenzoesäure, in eine epoxidische Verbindung übergeführt werden, die wiederum weiteren Reaktionen zugänglich ist. So kann sie in das Diol übergeführt werden, dessen OH-Gruppen zur Bildung von einfachen Estern oder Polyestern geeignet sind. Die erfindungsgemäße Umsetzung von teilweise fluorierten Alkenen mit Dienen, wie 1,6-Hexadien, führt zu oligomeren beziehungsweise polymeren Verbindungen, die mehrere Doppelbindungen aufweisen und elastomeren Charakter haben. Diese Elastomeren sind vernetzbar, wodurch wärmebeständige Kunststoffe entstehen.

Die erfindungsgemäße Umsetzung ermöglicht es, zu längerkettigen Verbindungen mit teilweise fluorierten Resten zu gelangen, die sonst nur über umständlichere Verfahrensweisen, die häufig mehrere Reaktionsschritte benötigen, zugänglich wären.

Nachfolgende Beispiele sollen die Erfindung erläutern.

### Beispiel 1

In einem Glaskolben von 10 ccm Inhalt werden unter trockenem Stickstoff 2 ccm (12 mmol) 4-(Perfluorisopropyl)-1-buten und 200 mg eines festen feinteiligen Katalysators gegeben, der durch Umsetzung eines festen Siliciumdioxid-Chrom(II)-Katalysators mit der Verbindung (CO)₄ClW≡C-Phenyl erhalten wurde, wie in "Journal of Organometallic Chemistry", 355 (1988), Seite 279 beschrieben. Der Katalysator enthält 0,022 mmol Wolfram-Verbindung. Auf 100 mol 4-(Perfluorisopropyl)-1-buten werden 0,18 mol Wolfram-Verbindung eingesetzt. Die Mischung wird 7 Stunden bei 76 °C geschüttelt, dann abgekühlt, mit 10 ccm Pentan vermischt und vom festen Katalysator abdekantiert. Der Katalysator wird mit weiteren 5 ccm Pentan geschüttelt, die Flüssigkeit erneut abdekantiert, mit der Hauptmenge vereinigt und über eine 1 cm hohe Schicht von feinteiligem Aluminiumoxid filtriert. Aus dem Filtrat wird bei 12 mbar (1,2 kPa) und 20 °C das Lösungsmittel und nicht umgesetztes 4-(Perfluorisopropyl)-1-buten abdestilliert. Der Rückstand wird bei normalem Atmosphärendruck destilliert, der Hauptteil geht bei 153 °C über und hat eine Dichte von 1,56 g/cm³. Mit der ¹³C- und ¹⁹F-Kernresonanzspektroskopie (NMR) in CDCl₃, bezogen auf Tetramethylsilan und Perfluoressigsäure, werden folgende Signale erhalten:
- ¹³C-NMR (ppm):: 121,4 CF₃; 91,3 CF; 28,9 CH₂; 24,6 und 19,5 CH₂-(C=); 129,5 und 128,8 -HC=CH-(cis, trans)
- ¹⁹F-NMR (ppm):: -1,84; -1,12 und -1,18 CF₃.

Wie die Analyse bestätigt, besteht der Hauptteil aus 1,6-Bis-(perfluorisopropyl)-3-hexan 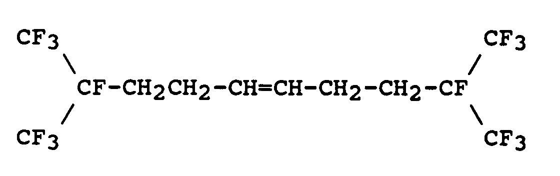
Es werden 1,9 g = 4,5 mmol = 75 % des theoretischen Wertes erhalten. (Die Ausbeute ist geringer als die gaschromatographisch ermittelte. Vermutlich ist durch die Filtration über Al₂O₃ ein Substanzverlust durch Absorption aufgetreten.)

### Beispiel 2

In einem Glaskolben von 20 ccm Inhalt werden unter trockenem Stickstoff 250 mg (0,028 mmol, bezogen auf Wolfram-Verbindung) des in Beispiel 1 beschriebenen festen Katalysators in 10 ccm Hexan bei Raumtemperatur suspendiert, auf 69 °C erwärmt, 1,3 ccm (5,54 mmol) der Verbindung C₆F₁₃CH₂-CH=CH₂ zugegeben und 7 Stunden bei 69 °C geschüttelt. Auf 100 mol C₆F₁₃CH₂CH=CH₂ werden 0,5 mol Wolfram-Verbindung eingesetzt. Während der Reaktion wird die Abnahme der Ausgangssubstanz und die Zunahme des Umsetzungsproduktes gaschromatographisch verfolgt. Das Umsetzungsprodukt wird durch gaschromatographische Analyse mit unmittelbar anschließender massenspektroskopischer Analyse (GC/MS-Analyse) als C₆F₁₃CH₂CH=CHCH₂C₆F₁₃ charakterisiert. Der Gesamtumsatz des Ausgangsproduktes beträgt nach 7 Stunden 93,5 % (auf 100 % möglichen Umsatz normiert). Je Stunde und je mol Wolfram-Verbindung werden 71,4 mol des Ausgangsalkens umgesetzt.

### Beispiel 3

Es wird analog Beispiel 2 gearbeitet, doch werden 220 mg (0,025 mmol, bezogen auf Wolfram-Verbindung) des in Beispiel 1 beschriebenen festen Katalysators ohne Lösungsmittel eingesetzt, 5,8 ml (24,4 mmol) C₆F₁₃CH₂CH=CH₂ zugegeben und 7 Stunden bei 122 °C geschüttelt. Auf 100 mol C₆F₁₃CH₂CH=CH₂ werden 0,1 mol Wolfram-Verbindung eingesetzt. Das Umsetzungsprodukt wird als C₆F₁₃CH₂CH=CHCH₂C₆F₁₃ charakterisiert. Der Gesamtumsatz des Ausgangsproduktes beträgt nach 7 Stunden 16,9 % (auf 100 % Umsatz normiert). Je Stunde und je mol Wolfram-Verbindung werden 160,0 mol des Ausgangsalkens umgesetzt.

### Beispiel 4

Es wird analog Beispiel 2 gearbeitet. 230 mg (0,026 mmol, bezogen auf Wolfram-Verbindung) des in Beispiel 1 beschriebenen festen Katalysators werden bei Raumtemperatur in 10 ccm Hexan suspendiert, auf 69 °C erwärmt, 1,3 ccm (5,1 mmol) der Verbindung C₆F₁₃CH₂CH₂CH=CH₂ zugegeben und 7 Stunden bei 69 °C geschüttelt. Auf 100 mol C₆F₁₃CH₂CH₂CH=CH₂ werden 0,5 mol Wolfram-Verbindung eingesetzt. Das Umsetzungsprodukt wird durch GC/MS-Analyse als C₆F₁₃CH₂CH₂CH=CHCH₂CH₂C₆F₁₃ charakterisiert. Der Gesamtumsatz des Ausgangsproduktes beträgt nach 7 Stunden 43,7 % (auf 100 % möglichen Umsatz normiert). Je Stunde und je mol Wolfram-Verbindung werden 37,6 mol des Ausgangsalkens umgesetzt.

### Beispiel 5

Es wird analog Beispiel 2 gearbeitet. 45 mg (0,1 mmol) der Verbindung (CH₂OCH₂CH₂OCH₃)Cl₃W≡C-C(CH₃)₃, hergestellt, wie beschrieben in "Organometallics", Vol. 1, Nr. 12 (1982), Seite 1 649, rechte Spalte werden in 10 ccm 1,2-Dichlorethan bei Raumtemperatur gelöst, 11,2 g (50 mmol) (CF₃)₂CF-CH₂CH₂-CH=CH₂ zugegeben und 24 Stunden bei 76 °C geschüttelt. Auf 100 mol (CF₃)₂CFCH₂CH₂CH=CH₂ werden 0,2 mol Wolfram-Verbindung eingesetzt. Das Umsetzungsprodukt wird durch GC/MS-Analyse als (CF₃)₂CFCH₂CH₂CH=CHCH₂CH₂CF(CF₃)₂ charakterisiert. Der Gesamtumsatz des Ausgangsproduktes beträgt nach 24 Stunden 63,4 % (auf 100 % möglichen Umsatz normiert). Je Stunde und je mol Wolfram-Verbindung werden 44 mol des Ausgangsalkens umgesetzt.

### Beispiel 6

Es wird analog Beispiel 2 gearbeitet. 150 mg (0,029 mmol, bezogen auf Wolfram-Verbindung) eines festen feinteiligen Katalysators, der durch Umsetzung eines festen Siliciumdioxid-Katalysators mit der Verbindung [(CH₃)₃C-CH₂-]₃W≡C-C(CH₃)₃ erhalten wurde, wie in "Angewandte Chemie", 101 (1989), Seite 76 beschrieben, werden in 10 ml Heptan bei Raumtemperatur suspendiert, auf 69 °C erwärmt, 1 ccm (5,78 mmol) der Verbindung (CF₃)₂CFCH₂CH₂CH=CH₂ zugegeben und 7 Stunden bei 69 °C geschüttelt. Auf 100 mol (CF₃)₂CFCH₂CH₂CH=CH₂ werden 0,5 mol Wolfram-Verbindung eingesetzt. Das Umsetzungsprodukt wird durch GC/MS-Analyse als (CF₃)₂CFCH₂CH₂CH=CHCH₂CH₂CF(CF₃)₂ charakterisiert. Der Gesamtumsatz des Ausgangsproduktes beträgt 88,1 % (auf 100 % möglichen Umsatz normiert). Je Stunde und je mol Wolfram-Verbindung werden 260,8 mol des Ausgangsalkens umgesetzt.

### Beispiel 7

Es wird gearbeitet, wie in Beispiel 6 beschrieben, mit dem Unterschied, daß kein Heptan verwendet wird und anstelle 1 ccm 5,1 ccm (28,9 mmol) der Verbindung (CF₃)₂CFCH₂CH₂CH=CH₂ eingesetzt werden, in denen der feste Katalysator suspendiert wird. Die Umsetzung wird während 7 Stunden bei 76 °C durchgeführt. Auf 100 mol der Verbindung (CF₃)₂CFCH₂CH₂CH=CH₂ werden 0,1 mol der Wolfram-Verbindung eingesetzt. Das Umsetzungsprodukt ist dasselbe wie in Beispiel 6; der Gesamtumsatz des Ausgangsproduktes beträgt nach 7 Stunden 94,4 % (auf 100 % Umsatz normiert). Je Stunde und je mol Wolfram-Verbindung werden 1 240 mol des Ausgangsalkens umgesetzt.

### Beispiel 8

Es wird analog Beispiel 2 gearbeitet. 240 mg (0,027 mmol, bezogen auf Wolfram-Verbindung) des in Beispiel 1 beschriebenen festen Katalysators werden bei Raumtemperatur in 4,7 ccm (26,6 mmol) der Verbindung (CF₃)₂CFCH₂CH₂CH=CH₂ suspendiert und die Mischung 7 Stunden bei 76 °C geschüttelt. Auf 100 mol (CF₃)₂CFCH₂CH₂CH=CH₂ werden 0,1 mol Wolfram-Verbindung eingesetzt. Es entsteht dasselbe Umsetzungsprodukt wie in Beispiel 6. Der Gesamtumsatz des Ausgangsproduktes beträgt nach 7 Stunden 97 % (auf 100 % möglichen Umsatz normiert). Je Stunde und je mol Wolfram-Verbindung werden 1 570 mol des Ausgangsalkens umgesetzt.

### Beispiel 9

In einem Glaskolben von 20 ccm Inhalt werden 120 mg (0,023 mmol, bezogen auf Wolfram-Verbindung) des in Beispiel 6 beschriebenen festen Katalysators in 10 ccm Heptan bei 60 °C suspendiert, 0,54 ccm (4,6 mmol) 1,5-Hexadien und 0,82 ccm (4,6 mmol) 4-(Perfluorisopropyl)-1-buten zugegeben, die Mischung auf 69 °C erwärmt und 5 Stunden geschüttelt. Auf 100 mol 4-(Perfluorisopropyl)-1-buten werden 100 mol 1,5-Hexadien und auf 100 mol der Mischung beider genannten Ausgangsstoffe werden 0,25 mol Wolfram-Verbindung eingesetzt. Nach 5 Stunden wird das entstandene Reaktionsgemisch vom Katalysator getrennt und gaschromatographisch sowie gaschromatographisch mit unmittelbar anschließender Massenspektrographie (GC/MS-Analyse) untersucht, wobei folgende Werte ermittelt werden:

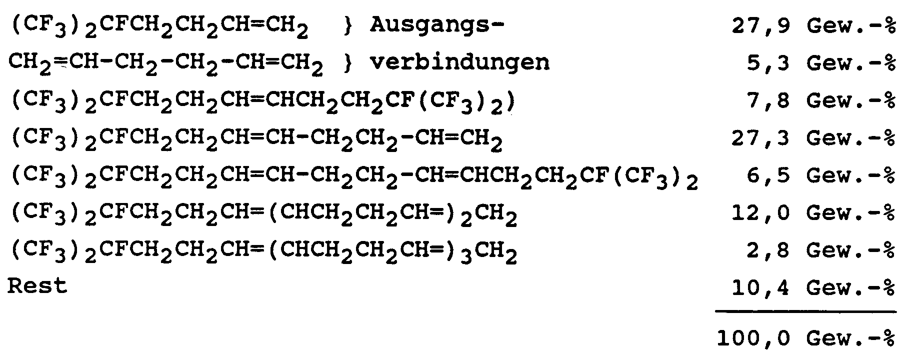

Der Rest besteht aus einem Gemisch folgender Verbindungen:
(CF₃)₂CFCH₂CH₂CH=(CHCH₂CH₂CH=)_{y}CHCH₂CH₂CF(CF₃)₂
y = 2 und 3
(CF₃)₂CFCH₂CH₂CH=(CHCH₂CH₂CH=)_{z}CH₂
z = 4, 5 und 6.

Wie ersichtlich sind längerkettige Alkene mit 1 bis 6 mittelständigen -CH=CH-Gruppen entstanden, die entweder an einem oder an beiden Kettenenden Perfluorisopropyl-Gruppen tragen.

### Beispiel 10

Es wird analog Beispiel 2 gearbeitet. 130 mg (0,026 mmol, bezogen auf die Wolfram-Verbindung) eines feinteiligen, festen Katalysators, der durch Umsetzung eines festen Siliciumdioxid-Chrom(II)-Katalysators mit der Verbindung (CO)₅W=C(Phenyl)₂ unter Abspaltung von CO erhalten wurde, wie in "Angewandte Chemie", 100 (1988), Seiten 268 bis 270 beschrieben, werden in 4 ccm Heptan bei Raumtemperatur suspendiert und 0,9 ccm (5,2 mmol) 4-Perfluorisopropyl-1-buten zugegeben. Das Gemisch wird unter Rühren auf 76 °C erwärmt. Auf 100 mol 4-Perfluorisopropyl-1-buten werden 0,5 mol Wolfram-Verbindung eingesetzt. Während der Reaktion wird die Abnahme der Ausgangssubstanz und die Zunahme des Umsetzungsproduktes gaschromatographisch verfolgt. Nach 1 Stunde Reaktionszeit beträgt der Umsatz 26,1 Gew.-%, dies entspricht einer Umsatzgeschwindigkeit von 52 mol Perfluoralkylalken je mol der Wolfram-Verbindung. Nach 24 Stunden Reaktionszeit beträgt die Gesamtausbeute 55 Gew.-% (auf 100 % mögliche Ausbeute normiert).

## Patentansprüche

1. Verfahren zur Umsetzung von substituierten Kohlenwasserstoffen, die mindestens eine Doppelbindung aufweisen, mit Wolfram enthaltenden Katalysatoren unter Abspaltung von Ethylen oder einem Alken und Bildung längerkettiger substituierter Kohlenwasserstoffe, die mindestens eine mittelständige Doppelbindung aufweisen, bei 10 bis 200 °C, falls erforderlich in Gegenwart eines inerten Lösungsmittels, unter normalem Atmosphärendruck, vermindertem Druck oder dem autogenen Druck der Reaktionsmischung, dadurch gekennzeichnet, daß zur Umsetzung mindestens ein Alken der Formeln
X-R_{f}-(CH₂)ₙCH=CH-Z (I)
oder
CH₂F-(CH₂)ₘ-CH=CH-Z (II)
eingesetzt wird, in denen bedeuten
R_{f} ein geradkettiger oder verzweigter oder ringförmiger Perfluoralkylenrest mit 1 bis 18 C-Atomen,
m und n je eine ganze Zahl von 1 bis 10,
X = F, H, Cl, Alkyl mit 1 bis 6 C-Atomen oder Aryl mit 6 bis 10 C-Atomen und
Z = H, Alkyl mit 1 bis 10 C-Atomen, Arylalkyl mit 7 bis 11 C-Atomen, den Rest -(CH₂)ₙR_{f}X oder den Rest -(CH₂)ₘ-CH₂F, wobei, wenn nur eine Verbindung eingesetzt wird, diese unterschiedliche Substituenten auf beiden Seiten der -CH=CH-Gruppe aufweisen muß.

2. Verfahren nach Anspruch 1, dadurch gekennzeichnet, daß zusätzlich zu mindestens einer Verbindung der Formeln (I) oder (II) mindestens eine Verbindung der Formel
A-(CH₂)ₜ-CH=CH-E (III)
eingesetzt wird, in der bedeuten
A = H, -CH₂Cl, Aryl mit 6 bis 10 C-Atomen oder -CH=CH₂,
t = eine ganze Zahl von 1 bis 10 und
E = H, Alkyl mit 1 bis 10 C-Atomen oder Arylalkyl mit 7 bis 11 C-Atomen,
die Bindungen bei A und E können zu einem Ring geschlossen sein.

3. Verfahren nach Anspruch 1 oder 2, dadurch gekennzeichnet, daß der Katalysator aufgebaut ist aus einem festen Träger, der im wesentlichen aus Siliciumdioxid, Aluminiumoxid oder Mischungen dieser beiden Oxide besteht, an den über O-Atome oder über O-Atome und Chrom das Wolfram als eine Komplexverbindung, die eine Bindung zwischen Wolfram und Kohlenstoff aufweist, gebunden ist.

4. Verfahren nach Anspruch 1 oder 2, dadurch gekennzeichnet, daß ein Katalysator der Formel 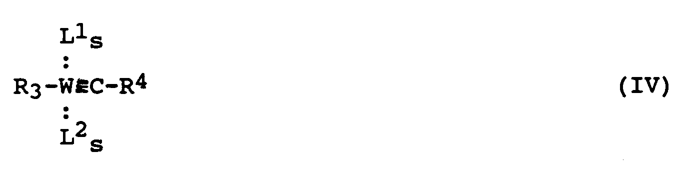 eingesetzt wird, in der bedeuten
R = Cl, -CH₂-C(CH₃)₃, -O-C(CH₃)₃, -S-C(CH₃)₃ oder -NG₂, worin G eine Alkylgruppe mit 1 bis 4 C-Atomen oder der Benzylrest ist,
R⁴ = eine geradkettige, verzweigte oder cyclische Alkylgruppe mit 1 bis 6 C-Atomen, eine Trialkylsilylgruppe, deren Alkylreste je 1 bis 3 C-Atome enthalten, eine Benzyl-, Phenyl-, Tolyl- oder Naphthylgruppe,
L¹ = ein Trialkylphosphin, Trialkylphosphinoxid oder Ethylenglykoldialkylether, deren Alkylgruppen 1 bis 4 C-Atome aufweisen,
L² = ein Trialkylphosphin, Trialkylphosphinoxid, Trialkylphosphoniumchlorid oder Tetraalkylammoniumchlorid, deren Alkylgruppen 1 bis 4 C-Atome aufweisen, mit der Maßgabe, daß R = Cl ist, wenn L² ein Trialkylphosphonium oder Tetraalkylammoniumchlorid ist, und
s = Null oder 1.

5. Verfahren nach einem oder mehreren der Ansprüche 1 bis 4, dadurch gekennzeichnet, daß mindestens eine Verbindung der Formel (I) eingesetzt wird, in der X = F und Z = H ist.

6. Verfahren nach einem oder mehreren der Ansprüche 2 bis 5, dadurch gekennzeichnet, daß mindestens eine Verbindung der Formel (II) eingesetzt wird, in der A = -CH=CH₂ und E = H ist.

## Claims

1. A process for the reaction of a substituted hydrocarbon which contains at least one double bond using a tungsten-containing catalyst, ethylene or an alkene being split off, to form a longer-chain substituted hydrocarbon which contains at least one central double bond, at 10 to 200°C, if necessary in the presence of an inert solvent, under normal atmospheric pressure, reduced pressure or the autogenous pressure of the reaction mixture, which comprises using, for the reaction, at least one alkene of the formula
X-R_{f}-(CH₂)ₙ-CH=CH-Z (I)
or
CH₂F-(CH₂)ₘ-CH=CH-Z (II)
in which
R_{f} is a straight-chain, branched or cyclic perfluoroalkylene radical having 1 to 18 carbon atoms,
m and n are each an integer from 1 to 10,
X is F, H, Cl, alkyl having 1 to 6 carbon atoms or aryl having 6 to 10 carbon atoms and
Z is H, alkyl having 1 to 10 carbon atoms, arylalkyl having 7 to 11 carbon atoms, the radical -(CH₂)ₙR_{f}X or the radical -(CH₂)ₘ-CH₂F, in which, if only one compound is employed, this must have different substituents on the two sides of the -CH=CH- group.

2. The process as claimed in claim 1, wherein, in addition to at least one compound of the formula (I) or (II), at least one compound of the formula
A-(CH₂)ₜ-CH=CH-E (III)
in which
A is H,-CH₂Cl, aryl having 6 to 10 carbon atoms or -CH=CH₂,
t is an integer from 1 to 10 and
E is H, alkyl having 1 to 10 carbon atoms or arylalkyl having 7 to 11 carbon atoms,
and the bonds at A and E can be closed to form a ring,
is employed.

3. The process as claimed in claim 1 or 2, wherein the catalyst is built up from a solid support, which essentially consists of silicon dioxide, aluminum oxide or a mixture of these two oxides, onto which tungsten is bonded, via O atoms or via O atoms and chromium, as a complex compound which has a bond between tungsten and carbon.

4. The process as claimed in claim 1 or 2, wherein a catalyst of the formula 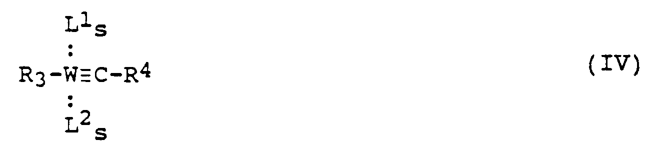 in which
R is Cl, -CH₂-C(CH₃)₃, -O-C(CH₃)₃, -S-C(CH₃)₃ or -NG₂, in which G is an alkyl group having 1 to 4 carbon atoms or the benzyl radical,
R⁴ is a straight-chain, branched or cyclic alkyl group having 1 to 6 carbon atoms, a trialkylsilyl group, the alkyl radicals of which each comprise 1 to 3 carbon atoms, or a benzyl, phenyl, tolyl or naphthyl group,
L¹ is a trialkylphosphine, trialkylphosphine oxide or ethylene glycol dialkyl ether, the alkyl groups of which contain 1 to 4 carbon atoms,
L² is a trialkylphosphine, trialkylphosphine oxide, trialkylphosphonium chloride or tetraalkylammonium chloride, the alkyl groups of which contain 1 to 4 carbon atoms, with the proviso that R is Cl if L² is a trialkylphosphonium or tetralkylammonium chloride, and
s is zero or 1,
is employed.

5. The process as claimed in one or more of claims 1 to 4, wherein at least one compound of the formula (I) in which X is F and Z is H is employed.

6. The process as claimed in one or more of claims 2 to 5, wherein at least one compound of the formula (II) in which A is -CH=CH₂ and E is H is employed.

## Revendications

1. Procédé pour faire réagir des hydrocarbures substitués, qui comportent au moins une double liaison, avec des catalyseurs contenant du tungstène, avec élimination d'éthylène ou d'un alcène et formation d'hydrocarbures substitués à chaîne plus longue, qui comportent au moins une double liaison en position centrale, à une température de 10 à 200°C, si nécessaire en présence d'un solvant inerte, sous la pression atmosphérique normale, sous une pression réduite ou sous la pression spontanée du mélange réactionnel, caractérisé en ce que l'on utilise pour la réaction au moins un alcène de formules :
X-R_{f}-(CH₂)ₙ-CH=CH-Z (I)
ou
CH₂F-(CH₂)ₘ-CH=CH-Z (II)
dans lesquelles :
R_{f} est un radical perfloroalkylène à chaîne droite ou ramifiée, ou cyclique, ayant de 1 à 18 atomes de carbone,
m et n sont chacun un nombre entier de 1 à 10,
X est F, H, Cl, un radical alkyle ayant de 1 à 6 atomes de carbone ou aryle ayant de 6 à 10 atomes de carbone, et
Z est H ou un radical alkyle ayant de 1 à 10 atomes de carbone, arylalkyle ayant de 7 à 11 atomes de carbone, le radical -(CH₂)ₙR_{f}X ou le radical -(CH₂)ₘ-CH₂F, où, quand on utilise un seul composé, ce dernier doit comporter des substituants différents sur les deux côtés du groupe -CH=CH-.

2. Procédé selon la revendication 1, caractérisé en ce que, en plus d'au moins un composé de formules (I) ou (II), on utilise au moins un composé de formule :
A-(CH₂)ₜ-CH=CH-E (III)
dans laquelle :
A est H, -CH₂Cl ou un radical aryle ayant de 6 à 10 atomes de carbone, ou -CH=CH₂,
t est un nombre entier de 1 à 10, et
E est H ou un radical alkyle ayant de 1 à 10 atomes de carbone ou arylalkyle ayant de 7 à 11 atomes de carbone,
les liaisons en A et E peuvent être fermées pour former un cycle.

3. Procédé selon la revendication 1 ou 2, caractérisé en ce que le catalyseur est constitué d'un support solide qui pour l'essentiel est constitué de dioxyde de silicium, d'oxyde d'aluminium ou de mélanges de ces deux oxydes, auquel cas, par l'intermédiaire d'atomes d'oxygène ou par l'intermédiaire d'atomes d'oxygène et de chrome, le tungstène est lié sous forme d'un composé complexe, qui comporte une liaison entre le tungstène et le carbone.

4. Procédé selon la revendication 1 ou 2, caractérisé en ce qu'on utilise un catalyseur de formule : 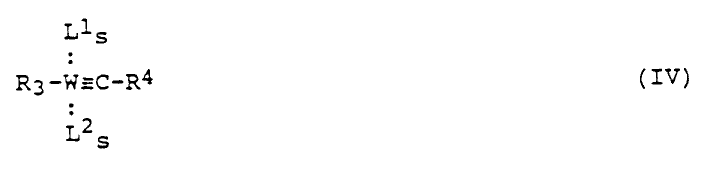 dans laquelle :
R est Cl, -CH₂-C(CH₃)₃, -O-C(CH₃)₃, -S-C(CH₃)₃ ou -NG₂, où G est un groupe alkyle ayant de 1 à 4 atomes de carbone ou le radical benzyle,
R⁴ est un groupe alkyle à chaîne droite ou ramifiée ou cyclique, ayant de 1 à 6 atomes de carbone, un groupe trialkylsilyle, dont les fragments alkyle contiennent chacun de 1 à 3 atomes de carbone, un groupe benzyle, phényle, tolyle ou naphtyle,
L¹ est une trialkylphosphine, un oxyde de trialkylphosphine ou un éther dialkylique de l'éthylèneglycol, dont les fragments alkyle contiennent de 1 à 4 atomes de carbone,
L² est une trialkylphosphine, un oxyde de trialkylphosphine, un chlorure de trialkylphosphonium ou un chlorure de tétraalkylammonium, dont les fragments alkyle ont de 1 à 4 atomes de carbone, du moment que, quand R est Cl, alors L² est un chlorure de trialkylphosphonium ou un chlorure de tétraalkylammonium, et
s vaut 0 ou 1.

5. Procédé selon une ou plusieurs des revendications 1 à 4, caractérisé en ce qu'on utilise au moins un composé de formule (I) dans laquelle X est F et Z est H.

6. Procédé selon une ou plusieurs des revendications 2 à 5, caractérisé en ce qu'on utilise au moins un composé de formule (II) dans laquelle A est -CH=CH₂ et E est H.
